(19) Europäisches Patentamt
European Patent Office
Office européen des brevets

(11) Publication number: **0 004 681 B1**

(12) # EUROPEAN PATENT SPECIFICATION

(45) Date of publication of patent specification: **25.05.83**

(21) Application number: **79101143.0**

(22) Date of filing: **12.04.79**

(51) Int. Cl.[3]: **C 09 B 61/00, C 07 D 311/40, C 07 D 311/62**

(54) **Process for preparing anthocyans from the corresponding flavonoid glycosides.**

(30) Priority: **12.04.78 JP 43700/78**

(43) Date of publication of application: **17.10.79 Bulletin 79/21**

(45) Publication of the grant of the patent: **25.05.83 Bulletin 83/21**

(84) Designated Contracting States: **BE CH DE FR GB IT LU NL SE**

(56) References cited:
**DE - A - 2 703 375**

**Chemical Abstracts, Volume 86, Nr. 17 1977, (COLUMBUS, OHIO, USA) K. WAKIHIRA et al. "Anthocyans" page 582, column 1, abstract Nr. 121704s**

**Chemical Abstracts, Volume 52, Nr. 6, 1957, (COLUMBUS, OHIO, USA) H. G. C. KING et al. "Conversion of flavanols into anthocyanidins" column 4612 c-d**

**The file contains technical information submitted after the application was filed and not included in this specification**

(73) Proprietor: **SHIRAIMATSU SHINYAKU COMPANY LTD.**
**37-1, Aza-Inaba**
**O'aza-Ukawa, Minakuchi-cho Koka-gun, Shiga-ken (JP)**

(72) Inventor: **Wakihira, Kazuo**
**620-23, Kibugawa Minakuchi-cho**
**Koka-gun Shiga-ken (JP)**
Inventor: **Kikumoto, Satoshi**
**610-11, Kibugawa Minakuchi-cho**
**Koka-gun Shiga-ken (JP)**
Inventor: **Kigawa, Hiroshi**
**2-2, Joto Minakuchi-cho**
**Koka-gun Shiga-ken (JP)**
Inventor: **Nozaki, Osamu**
**1145, Iwane-higashi Kosei-cho**
**Koka-gun Shiga-ken (JP)**
Inventor: **Minami, Michio**
**537-3, Yoshimi-cho**
**Moriyama-shi Shiga-ken (JP)**

(74) Representative: **Vossius Vossius Tauchner Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 004 681 B1

Process for preparing anthocyans from the corresponding flavonoid glycosides

Anthocyans are becoming increasingly important as non-toxic food dyestuffs since many synthetic food dyestuffs have proved to be dangerous for humans.

The known processes for preparing anthocyans from flavonoid glycosides by reduction are either difficult or give poor yields.

This invention relates to an improved process for preparing anthocyans by reducing their corresponding flavonoid glycosides with a metal and an acid in a polar solvent which comprises:

(a) conducting the reduction reaction at not more than 30°C under acidic conditions, the reducing agent for flavonoid glycosides being a combination of magnesium and hydrogen chloride, or a combination of zinc and hydrogen chloride, and

(b) precipitating the anthocyans as their metal salts by neutralizing the reaction solution at 0 to 10°C with aqueous ammonia, adding a hydrophilic solvent and

(c) isolating the anthocyans from their corresponding metal salts by decomposition of the metal salts using hydrogen chloride and a polar organic solvent.

The process of this invention provides highly purified anthocyans by a relatively simple route in good yields without causing destruction of their glycoside structure.

Anthocyans (Anthocyanins) are very unstable pigments found in the flowers, fruits and leaves of plants and have in common the basic structure represented by the following formula:

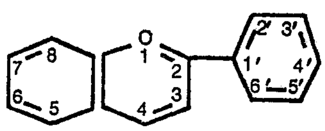

Processes for preparing anthocyans are roughly classificable into: 1) extraction from plants, 2) total synthesis from phenols or aromatic aldehydes and 3) removal of an oxygen atom from the 4-position and flavonoid glycosides by reduction. Of these processes, 1) requires a complex step for isolating and refining anthrocyans and 2) requires many steps resulting in high costs if purified products are to be provided, 3) is attainable in one relatively simple step, thus it has been the object of various investigations.

The known reductions of flavonols were conducted, for example, by employing a metal and an acid or sodium amalgam or lithium aluminium hydride; however, the employment of sodium amalgam, a mercury compound, is undesirable in view of the high toxicity of mercury, and various undesirable 4-oxy-flavones are obtained during the reduction process than the expected flavylium salt. The use of lithium aluminium hydride gives an average yield of 29%; it is however, necessary to convert the starting flavonol into a derivative soluble in an organic solvent.

With regard to the reduction of flavonols with a metal and an acid, it has been reported that pelargonidin chloride was obtained from kaemferol by a reductive acetylation with zinc dust which resulted in a yield of 40.8% by spectroscopic determination. However, no isolation step thereof was reported. Further a hydrolyzing step for removing acetyl groups was required for obtaining a free form of anthocyan in that reduction reaction.

As regards the reduction of flavonols with magnesium and hydrogen chloride, it was reported that isolation of anthocyans was carried out after the complex measures to isolate and purify some ingredients from plant bodies. This was accomplished, for example, via a lead salt or a salt of picric acid purifying agent or by chromatography. Nevertheless, the yield was not more than 2%.

C.A. *86,* 12170s (1977) corresponding to JP—A—76121034 discloses a preparation of pure anthocyans by reduction of flavonols or their glycosides under light irradiation in the presence of specific metals and acids.

C.A. *52,* 4612 c-d (1957), 4612 c-d discloses a method of converting flavonols into anthocyanidins.

Finally, in DE—A 2 703 375 the preparation of flavylium chlorides from their corresponding hydroxy flavones or their glycosides is disclosed. This process involves the following steps:

1. Reduction of the flavone or the corresponding glycoside derivative with magnesium and with sulfuric acid in a solvent;
2. Addition of dilute ammonia to the acidic solution until the pH reaches a value between 4 and 4.5;
3. Filtering off mineral salts which have been precipitated and washing with a solvent;
4. Concentration of the filtrate and extraction with ethyl acetate to remove impurities;
5. Adsorbing the purified aqueous solution on a resin, whereupon the resin is eluted with distilled water to remove the mineral salts;
6. Elution of the product from the resin with a mixture of HC1 and methanol;
7. Concentration of the eluate to dryness, to give the objective flavylium chloride.

Unlike this process, the process of the invention is characterized in its second step by precipitation of the anthocyans as their metal salts by neutralizing the reaction solution with aqueous ammonia. It has been found that this is a simple and effective way of obtaining the product in high purity compared with the known process in which the products require further purification.

In the process of the invention a flavonoid glycoside is dissolved in a polar solvent and a metal selected from the group of magnesium

and zinc is added. An acid, namely concentrated hdyrochloric acid is added to the solution at 20—30°C while stirring. Care must be taken to ensure that the solution is always kept acidic and that the temperature does not exceed 30°C. If the temperature rises above 30°C, hydrolysis of the glycoside occurs causing a decrease in the anthocyan yield. After addition of a prescribed quantity of the acid, stirring is continued until the reduction is complete. The reaction solution is then neutralized with aqueous ammonia, at temperatures of 0 to 10°C. A hydrophylic solvent, such as acetone, is added, whereupon the anthocyan precipitates as a metal salt, which is collected. The collected product is treated with concentrated hydrochloric acid at a temperature below room temperature while stirring, liberating the free anthocyan from its salt. The anthocyan is isolated and washed with dilute hydrochloric acid and is recrystallized from water or a polar organic solvent and hydrogen chloride.

The anthocyan thus obtained is identified by TLC or paper chromatography and found not to include organic impurities. It includes not more than 30 ppm of metallic impurities, such as magnesium. To ensure good yields it is convenient to use aqueous ammonia as a base. It has been found that dilute sulfuric acid, which was used for the decomposition of metal salts of anthocyans, is likely to cause hydrolysis of the anthocyans. Acetic acid and oxalic acid give poor yields of the objective anthocyans. It was found that hydrogen chloride is the most suitable acid for the present purpose, i.e. converting the metal salt into the free anthocyan.

The average yield obtained by the present process is 10—20%, which is far better than the yields obtained by the known processes.

The following specific examples serve to illustrate the present invention.

Example 1

20 g of rutin were dissolved in 200 ml of methanol and 10 g of magnesium were added. 100 ml of concentrated hydrogen chloride were added to the solution over a period of approximately one hour. The reaction temperature was maintained at 20—30°C by cooling with a mixture of ice and sodium chloride. After the addition was completed, the reaction mixture was stirred for 30 minutes. Then 36 ml of concentrated aqueous ammonia were dropped at 0—10°C into the reaction mixture under stirring, and the solution turned blue. 600 ml of acetone were added to the blue solution and a magnesium salt of *keracyanin* precipitated, which was collected by filtration. The collected material was added to 50 ml of concentrated hydrogen chloride, which was stirred for 3 hours at room temperature and was finally filtered. The material collected by filtration was again dispersed in 18% hydrogen chloride and was then again collected. The collected material was dissolved in 25 ml of dimethylformamide and was filtered 15 minutes later. 60 ml of concentrated hydrogen chloride were slowly added to the filtrate at 0—10°C. The reaction mixture was stirred for one hour and the precipitated keracyanin was collected by filtration and washed with dilute hydrogen chloride and dried under reduced pressure in a dessicator. The yield was 2.5 g (12.5%).

Example 2

200 g of myricitrin were dissolved in 2000 ml of methanol and 100 g of magnesium were added. One litre of concentrated hydrogen chloride was added to the solution over a period of approximately 2 hours. The reaction temperature was maintained at 20—30°C by cooling with a mixture of ice and sodium chloride. After the addition the reaction mixture was stirred for about one hour and was cooled to 0—10°C. 300 ml of concentrated aqueous ammonia were dropped into the reaction mixture under stirring and 6 litres of acetone were added. The precipitate formed was collected by filtration and dispersed in 500 ml of cooled concentrated hydrogen chloride and stirred for 3 hours at room temperature. Then the precipitate was collected. The collected material was again dispersed in one litre of dilute hydrogen chloride and the mixture was stirred for one hour at 20—30°C and filtered. The material collected by filtration was dissolved in 230 ml of dimethylformamide and the solution was filtered. The filtrate was added to concentrated hydrogen chloride, the amount of which was 1.5 times the amount of the filtrate. The resulting mixture was stirred for 2 hours, whereupon deposition occured. The keracyanin thus precipitated was collected and washed first with dilute hydrogen chloride and then with a mixture of acetone and ethyl acetate and was dried in a dessicator. The yield was 30 g (15%).

**Claim**

A process for preparing anthocyans by reducing their corresponding flavonoid glycosides with a metal and an acid in a polar solvent which comprises:

(a) conducting the reduction reaction at not more than 30°C under acidic conditions, the reducing agent for flavonoid glycosides being a combination of magnesium and hydrogen chloride or a combination of zinc and hydrogen chloride, and

(b) precipitating the anthocyans as their metal salts by neutralizing the reaction solution at 0 to 10°C with aqueous ammonia, adding a hydrophilic solvent and

(c) isolating the anthocyans from their corresponding metal salts by decomposition of the metal salts using hydrogen chloride and a polar organic solvent.

**Revendication**

Un procédé de préparation d'anthocyanes par réduction de leurs glucosides de flavonoïdes correspondants avec un métal et un acide, dans un solvant polaire, qui consiste

(a) à mener la réaction de réduction à une température non supérieure à 30°C sous des conditions acides, l'agent réducteur pour les glucosides de flavonoïdes étant une combinaison de magnésium et d'acide chlorhydrique ou une combinaison de zinc et d'acide chlorhydrique et

(b) à faire précipiter les anthocyanes sous forme d'un sel métallique par neutralisation de la solution de réaction à 0—10°C avec de l'ammoniac aqueux, en ajoutant un solvant hydrophile et

(c) en isolant les anthocyanes à partir de leurs sels métalliques correspondants par décomposition des sels métalliques en utilisant de l'acide chlorhydrique et un solvant organique polaire.

**Patentanspruch**

Verfahren zur Herstellung von Anthocyanen durch Reduktion ihrer entsprechenden Flavonoidglykoside mit einem Metall und einer Säure in einem polaren Lösungsmittel, dadurch gekennzeichnet, daß

(a) die Reduktionsreaktion bei Temperaturen bis zu 30°C im sauren Milieu durchgeführt wird, das Reduktionsmittel für Flavonoidglykoside eine Kombination aus Magnesium und Chlorwasserstoff oder aus Zink und Chlorwasserstoff ist, und

(b) die Anthocyane als Metallsalz durch neutralisation der Reaktionslösung bei 0 bis 10°C mit wässrigem Ammoniak und Zusatz eines hydrophilen Lösungsmittels ausgefällt und

(c) durch Zersetzung der Metallsalze unter Verwendung von Chlorwasserstoff und einem polaren organischen Lösungsmittel isoliert werden.